# EUROPEAN PATENT SPECIFICATION

(11) **EP 3 750 411 B1**
(45) Date of publication and mention of the grant of the patent: **18.12.2024**
(21) Application number: 20020275.2
(22) Date of filing: 11.06.2020
(51) Int. Cl.: A23L 33/16, A23L 33/18, A23L 33/19, A61P 17/02, A23K 50/40, A23K 20/147, A23K 20/20, A61P 19/02

(54) **NUTRITIONAL SUPPLEMENT FOR THE ENHANCED HEALING OF INJURIES**
NAHRUNGSERGÄNZUNGSMITTEL ZUR VERBESSERTEN HEILUNG VON VERLETZUNGEN
COMPLÉMENT ALIMENTAIRE POUR AMÉLIORER LA GUÉRISON DE BLESSURES

(30) Priority: 12.06.2019 BE 201900049
(43) Date of publication of application: 16.12.2020
(73) Proprietor: Vanlommel, Maria Kristel, 3980 Tessenderlo (BE)
(72) Inventor: Vanlommel, Florent, BE-3980 Tessenderlo (BE)
(74) Representative: Theunis, Patrick

(56) References cited:
- WO-A1-01/32188
- WO-A1-2007/145520
- WO-A1-2008/154178
- WO-A1-2015/177309
- CN-A- 110 771 741
- CN-A- 110 859 247
- US-A1- 2020 069 776
- DATABASE MEDLINE [online] US NATIONAL LIBRARY OF MEDICINE (NLM), BETHESDA, MD, US; November 1999 (1999-11-01), ZHOU Y ET AL: "[Glutamine dipeptide enriched enteral nutrition improving gut permeability in sever burns].", Database accession no. NLM11715489
- ZHOU Y ET AL: "[Glutamine dipeptide enriched enteral nutrition improving gut permeability in sever burns].", ZHONGHUA YI XUE ZA ZHI NOV 1999, vol. 79, no. 11, November 1999 (1999-11-01), pages 825 - 827, ISSN: 0376-2491

## Description

### Field of the invention:

The present invention relates to a nutritional supplement. More in particular, the present invention relates to a nutritional supplement composed of natural organic and inorganic food components. The composition activates the transformation of raw materials and their absorption in the metabolism.

It relates to a mixture with coordinating characteristics. The composition increases the efficiency of absorption of the raw materials incorporated in the formulation but also the effectiveness of other food ingredients, including medication.

### Background prior art of the invention

It is known today that numerous nutritional supplements for a large variety of aims and applications are available on the market. For example, there are formulations available, such as probiotics, that claim to significantly increase the healing process of wounds such as bone fractures, hip dysplasia, rheumatic conditions and others. This increased recovery is possible due to the improved intake and absorption of medication and food components. WO2008/154178A1 discloses a dietary supplement having a balanced mixture of compounds that promote the growth, repair, and maintenance of mammalian bone and joint connective tissue.

However, in reality, most of these supplements do not meet the expectations or have only a limited or no effectiveness.

As a result, there remains a need for a nutritional supplement with none of the abovementioned disadvantages and that generally meets the expectations of the user in a user-friendly manner, as indicated in the field of the invention.

### Summary of the invention

Consequently, the present invention aims to solve among others the abovementioned disadvantages by providing a nutritional supplement that is absorbed fast and effectively by the human metabolism, which as a result substantially increases the general recovery or healing process of injuries, and/or contributes to an improved mobility in general.

To this end, the present invention relates to a nutritional supplement that includes at least the following 5 ingredients or raw materials:
- Glutamine peptide;
- Lactoferrin;
- Milk salts;
- Tricalcium phosphate and
- Sodium acid pyrophosphate.

According to a preferred embodiment of the invention, the nutritional supplement contains the abovementioned ingredients in the amounts indicated below, expressed as weight parts:
- Glutamine peptide from 500 to 600 parts by weight;
- Lactoferrin from 10 to 20 parts by weight;
- Milk salts from 450 to 550 parts by weight;
- Tricalcium phosphate from 50 to 150 parts by weight, and
- Sodium acid pyrophosphate from 10 to 30 parts by weight.

The nutritional supplement of the invention further includes magnesium citrate.

According to a further preferred embodiment of the invention, the nutritional supplement contains next to the abovementioned ingredients, one or more of the following components: sodium bicarbonate, ferrous gluconate, isomaltulose, dextrose, vanilla, wheat protein, vitamin C, collagen peptide, MSM, glucosamine and/or orange aroma.

Finally, according to a further preferred embodiment of the invention, the nutritional supplement contains next to the abovementioned ingredients, one or more of the following components: Q10, chondroitin, hyaluronic acid, caffeine and/or taurine.

The nutritional supplement according to the present invention, can be efficiently and effectively used to accelerate recovery or healing from injuries, as mentioned above, and/or it can be used to improve the overall mobility of persons or animals. More in particular, the present invention includes the compositions and uses as set forth in the appended claims.

### Detailed description of the invention

The invention, as indicated above, relates to a nutritional supplement that at least comprises the following ingredients, components or raw materials:
- Glutamine peptide;
- Lactoferrin;
- Milk salts;
- Tricalcium phosphate;
- Sodium acid pyrophosphate and
- magnesium citrate.

In the description below each of these essential ingredients is discussed in more detail.

### 1. Glutamine peptide

Glutamine peptide is an organic opponent of glutamic acid and increases substantially the absorption capacity for glutamic acid. The peptide form is essential for muscle growth and wound healing. This component is a substantial and essential ingredient of the nutritional supplement according to the present invention.

Glutamic acid as such is the most important amino acid in the human body. However, this acid has a low absorption by itself. Moreover, this component is subjected to the so-called law of minimum. This implies that if an important, essential amino acid is lacking, glutamic acid will not be absorbed. It will transform into the missing amino-acid due to its special function. This keeps the activity of the amino acids in the body optimal and consequently makes glutamic acid essential to the human body. In the body it plays an important role in the development and maintenance of muscles and tendons.

This product can be purchased among others from the company Friesland Campina Domo, Stationsplein 4, Amersfoort, The Netherlands under the commercial name "Hyvital^{®} Wheat Glutamine PU". It relates to an enzymatic hydrolysed wheat protein that under strictly regulated conditions is prepared by using enzymes suitable for use in food applications. It contains a high level of peptide-bound glutamine.

### 2. Lactoferrin

Lactoferrin is an anti-inflammatory protein that belongs to the humoral component of the immune system of the body. Humoral components are enzymes in the body that decrease the impact of pathogens or activate other cells and enzymes who can destroy the pathogen.

Lactoferrin can be extracted from cow milk but is also found in high concentrations in human breast milk and horse milk.

For application in the current invention, lactoferrin can be extracted from the colostrum (first milk after birth of the calf) in which it is excreted after the birth of the calf. Colostrum is characterised by a large variety of protective features important for the further life of the calf among others the development of immunity against diseases. After several weeks the lactoferrin disappears from the milk.

The nutritional supplement in the present invention contains lactoferrin for its abovementioned beneficial characteristics but also and more importantly because lactoferrin contains an organic iron bond with the milk protein. Without limiting themselves to a scientific explanation for the effectiveness of the nutritional supplement according to this invention, the researchers are convinced that this iron in blood leads to extra transportation of oxygen whereby the glutamine peptide receives this extra oxygen in the tendons and bones of the recovering tissue. This leads to an increased supply of blood, and thus also oxygen, to the bones and tendons when injured. This will substantially enhance and/or accelerate, as mentioned above, the recovery or healing process.

This product is among others supplied by the company Friesland Campina Domo, Stationsplein 4, Amersfoort, The Netherlands under the commercial name "Domo^{®} Vivinal Lactoferrin TD". It relates to a natural milk glycoprotein which supports the human immune system and stimulates the protection against pathogens.

### 3. Milk salts

The term "milk salts" as used in the present invention and claims is understood to mean the mixture of milk salts that remain after butterfat and proteins are removed from milk. In conventional food this mixture of milk salts is used to prepare cheese, yoghurt or is used as an extra filling or smoothing component. Consequently, these milk salts relate to a mineral complex, extracted from milk, rich in calcium, phosphor and magnesium. It is for example supplied in micronized form as Lactoval HICAL (as described in more detail below) and contains mainly minerals of milk in the form of complexes between calcium and phosphate or citrate.

In particular, these milk salts as proposed in the formulation of the present invention are mainly calcium and phosphor mineral compounds. In the preferred embodiment of the present invention the total calcium percentage is not less than 25% and the total phosphor content is not less than 10% of the total mineral composition. An example of the composition of these milk salts minerals can be as follows: Calcium 26.5%, Phosphor 12.5%, Sodium 1.0%, Potassium 1.0%, Magnesium 0.9%.

The nutritional supplement according to the present invention contains this mixture of milk salts as it can be beneficially applied in the recovery process. The organic milk salts found in Lactoval, can regulate the acidity by buffering single acids such as lactic acid, oxalic acid and others and consequently these acids cannot exert adverse effect on the healing wound. Indeed, with wounds increasing acidity needs to be avoided as much as possible in muscles and joints.

The milk salts found in Lactoval relate to for example calcium, phosphor, sodium, potassium and magnesium on the one hand and lactose on the other. The neutralisation by these milk salts reduces or prevents the swelling of the wound and ameliorates the supply of glutamine peptide, as described above.

This product is offered among others by the company Friesland Campina Domo with headquarters at stationsplein 4, Amersfoort, The Netherlands and with registered office at NCB-laan 80, P.O. Box 13, 5460 BA Veghel, The Netherlands, under the commercial name "DMV ^{®} Lactoval ^{®} Hical". It relates to a calcium-rich mineral product derived from milk. It is extracted from milk by a unique isolation process. It contains standard milk ingredients such as milk minerals, milk protein and lactose. It is recommended to use for calcium strengthening in a broad gamma of food products. It contains milk mineral components, in particular in the form of calcium phosphate and citrate complexes.

Lactoval can be found on the market in two varieties: Lactoval Hical and Lactoval Hical micronized. They both contain approximately 26.5% calcium. Both varieties can be used in the nutritional supplement of the present invention.

### 4. Tricalcium phosphate

Traditionally tricalcium phosphate is used in food as an anti-caking agent in numerous powders. In the nutritional supplement according to the present invention tricalcium phosphate is also used as an anti-caking agent.

Not wishing to be bound by a scientific explanation, the researchers believe that the addition of tricalcium phosphate to the nutritional supplement according to the present invention mainly contributes by providing pentavalent phosphor. Pentavalent phosphor will continuously be transformed to divalent phosphor. This conversion will release a 5K caloric value. This extra caloric value creates an extra stimulus for the absorption of glutamine peptide by the body.

This product is supplied among others by the company Gadot Biochemical Industries Ltd, with headquarters at Hahistrudrut Ave 117, P.O. Box 10636, Haifa Bay, Israel, under the commercial name "GBi Tri Calcium Phosphate E-341".

### 5. Sodium acid pyrophosphate

In conventional food this ingredient is used as acid function for chemical yeast. The nutritional supplement according to this invention added sodium acid pyrophosphate to the formulation to maintain the continuous process of transformation of pentavalent phosphor to divalent phosphor. This will maintain the functioning of the energy source. Indeed, sodium acid pyrophosphate is a divalent phosphor which is insufficiently present in the body and consequently needs to be supplied preferably by intake via the nutritional supplement of the present invention.

### Weight ratios

The ingredients described above are the essential raw materials or components present in the nutritional supplement according to the present invention. Regarding the mutual weight ratios of these 5 ingredients: according to a preferred embodiment of the invention, the abovementioned ingredients are preferably present in the amounts indicated below, expressed as weight parts:
- Glutamine peptide neutral from 500 to 600 parts by weight;
- Lactoferrin from 10 to 20 parts by weight;
- Milk salts from 450 to 550 parts by weight;
- Tricalcium phosphate from 50 to 150 parts by weight and
- Sodium acid pyrophosphate from 10 to 30 parts by weight.

### Magnesium citrate

The nutritional supplement according to the present invention furthermore comprises magnesium citrate.

As conventional element in food, magnesium is added as extra mineral in for example vitamin supplements. In the nutritional supplement according to the present invention magnesium is used as an extra element to support the calcium intake. Indeed, the ratio calcium-to-magnesium needs to be 2/1 for optimal function.

Magnesium citrate, structural formula:

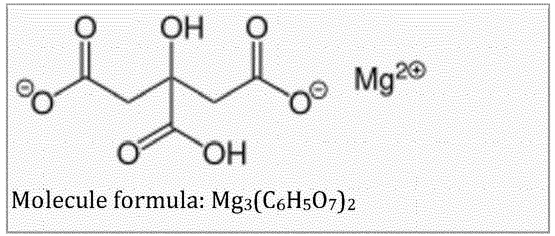

Magnesium citrate is the magnesium salt of citric acid and has the following bruto formula Mg₃(C₆H₅O₇)₂. However, in reality it only appears as a nona-hydrate Mg₃(C₆H₅O₇)₂ · 9 H₂O. It is a white, mild acid-tasting crystalline powder that contains 12% magnesium (according to weight). Magnesium citrate is easily prepared by reaction of magnesium hydroxide with citric acid.

This product can be purchased among others from the company Jungbunzlauer Ladenburg GmbH, Dr. Albert-Reimann-Str. 18, DE-68526 Landenburg, Duitsland under the commercial name "Trimagnesium Citrate Anhydrous USP Fine".

According to a further preferred embodiment of the invention, the nutritional supplement contains next to the abovementioned ingredients, one or more of the following components: sodium bicarbonate, ferrous gluconate, isomaltulose, dextrose, vanilla, wheat protein, vitamin C, collagen peptide, MSM, glucosamine and/or orange aroma.

A short description of the conventional function and the alleged contribution or function of the relevant ingredients in the nutritional supplement according to the present invention follows below.

### Sodium Bicarbonate

In conventional food this ingredient is used as basic function for chemical yeast or as raw material for all kind of preparations in the kitchen.

The nutritional supplement of the present invention uses sodium bicarbonate as a fast reaction to organic acids with expiring characteristics. After intake, this component will be completely metabolized and will thus disappear. This compound cannot be produced in the body and thus needs to be applied or absorbed on a daily basis.

### Ferrous gluconate

In conventional food this ingredient is used as an extra source of iron under an easily absorbable form. The nutritional supplement of the present invention uses ferrous gluconate as an extra source of iron to support the function of lactoferrin.

### Other possible ingredients:

Next to the abovementioned ingredients the nutritional supplement according to the present invention can contain one or more of the following components: isomaltulose, dextrose, vanilla, wheat protein, vitamin C, collagen peptide, MSM, glucosamine and/or orange aroma, Q10, chondroitin, hyaluronic acid, caffeine and/or taurine.

### MSM

The term "MSM" as used in the present description and conclusions is understood to be the abbreviation of Methyl Sulfonyl Methane and is a water-soluble component which appears in all life on earth such as plants, animals, humans, fish and algae. Sulfonyl points to a sulphur ingredient.

MSM is normally offered and available on the market in powder form. MSM powder contains a sulphur bond; sulphur is associated with different processes in the human body. It is an important element of the human body and lack of this chemical element leads to a reduced bodily function. MSM powder is a known additive to supplements containing glucosamine.

### Q10

The co-enzyme Q10 or simply Q10 in the description and claims of the present invention, is also known as ubichinon-10 and is a fat soluble, vitamin-like substance that is found in all human and animal cells. The most important function of this co-enzyme is serving as a co-factor in different important conversion steps part of the energy production (ATP-production) of the cell. It is also an important antioxidant. It is structurally related to vitamin K and vitamin E.

### Example

A concrete example of the formulation of the food ingredient or nutritional supplement according to the present invention contains the following components in the indicated weight percentages (expressed as parts by weight).

| | |
|---|---|
| - Glutamine peptide neutral | 500 |
| - Lactoferrin | 12 |
| - Milk salts | 510 |
| - Magnesium citrate | 110 |
| - Tricalcium phosphate | 90 |
| - Sodium acid pyrophosphate | 21 |
| - Sodium bicarbonate | 32 |
| - Ferric gluconate | 21 |
| - Isomaltulose | 200 |
| - Dextrose | 570 |
| - Vanilla | 14 |
| - Wheat protein | 400 |
| - Vitamin C | 100 |
| - Collagen peptide | 50 |
| - MSM | 35 |
| - Glucosamine | 30 |
| - Orange aroma | 50 |

A chemical analysis of this product gave the following result:

### Chemical Analysis

| | | | |
|---|---|---|---|
| Moisture | 5.8 | g/100g | based on ISO 1442^{a} |
| Ash | 19.7 | g/100g | based on ISO 936^{a} |
| Protein (N% x 6.25) | 29.7 | g/100g | ISO 1871^{a} |
| Fat after acid hydrolysis (Weibull) | 2.1 | g/100g | based on ISO 1443^{a} |
| Red. Sugars after inversion (as glucose) | 26.9 | g/100g | based on EG/152/2009^{a} |
| Dietary fibre (Asp) | 4.6 | g/100g | based on ASL00.00.18^{a} |
| Starch (EWERS) | 6.0 | g/100g | SM00120 |
| Total carbohydrates (calculated) | 38.1 | g/100g | SM00093 |
| Energy content | 299 | kcal/100g | SM00169 |
| Energy content | 1267 | kJ/100g | SM00169 |

In the table above, the first column shows the type of food component found in the nutritional supplement of the present invention, the second column shows the results of the investigation, the third column shows the unity of the results and the fourth column the applied measuring method.

Additionally, the fatty acid spectrum of the concerning product was determined on the total of all fatty acids present (C4-C22) in the nutritional supplement according to the invention.

### Fatty acid spectrum on total of fatty acids (C4-C22)

| | | | |
|---|---|---|---|
| C4:0 (butyric acid) | 0.9 | % | SM00716^{a} |
| C6:0 (Caproic acid) | 0.5 | % | SM00716^{a} |
| C8:0 (Caprylic acid) | 0.3 | % | SM00716^{a} |
| C10:0 (Capric acid) | 0.6 | % | SM00716^{a} |
| C12:0 (Lauric acid) | 1.2 | % | SM00716^{a} |
| C14:0 (Myristic acid) | 3.8 | % | SM00716^{a} |
| C14:1 (Myristoleic acid) | 0.3 | % | SM00716^{a} |
| C15:0 (Pentadecanoic acid) | 0.4 | % | SM00716^{a} |
| C16:0 (Palmitic acid) | 24.5 | % | SM00716^{a} |
| C16:1 (Palmitoleic acid) | 0.8 | % | SM00716^{a} |
| C17:0 (Heptadecanoic acid) | 0.2 | % | SM00716^{a} |
| C17:1 (Heptadecenoic acid) | 0.1 | % | SM00716^{a} |
| C18:0 (stearic acid) | 7.5 | % | SM00716^{a} |
| C18:1 (oleic acid) | 17.1 | % | SM00716^{a} |
| C18:2 (Linoleic acid) | 38.3 | % | SM00716^{a} |
| C18:3 (Linolenic acid) | 2.3 | % | SM00716^{a} |
| C20:0 (Arachidic acid) | 0.2 | % | SM00716^{a} |
| C20:1 (Eicosenoic acid) | 0.5 | % | SM00716^{a} |
| C20:2 (Eicosadienoic acid) | 0.1 | % | SM00716^{a} |
| C22:0 (Docosanoic acid) | 0.1 | % | SM00716^{a} |
| C22:1 (Docosenoic acid) | 0.1 | % | SM00716^{a} |
| C24:0 (Tetracosanoic acid) | 0.1 | % | SM00716^{a} |
| C24:1 (Tetracosenoic acid) | 0.1 | % | SM00716^{a} |
| Saturated fatty acid (total content) | 0.85 | % | SM00716^{a} |
| Mono-unsaturated fatty acid (total content) | 0.40 | % | SM00716^{a} |
| Poly-unsaturated fatty acids (total content) | 0.85 | % | SM00716^{a} |
| Trans fatty acids (Total content) | 0.027 | % | SM00716^{a} |
| Omega 3 fatty acids (Total content) | 0.048 | % | SM00716^{a} |
| Omega 6 fatty acids (Total content) | 0.80 | % | SM00716^{a} |
| Sodium | 0.91 | g/100g | Based on ASL07.00.56^{a} |

The columns 2,3 and 4 have the same meaning as in the previous table.

### Experimental support (I)

A veterinary surgeon has used the nutritional supplement of the present invention in order to boost or improve the general mobility of animals, dogs in particular, and has obtained excellent results, not only in cases of recovery or wound healing after surgery, but also to obtain a general functional recovery - e.g. from a reumatical disease - absent a prior surgical intervention.

The veterinary surgeon has obtained such beneficial results in particular with dogs, suffering from the following diseases: panosteitis, elbow dysplasis, osteochondrosis disease, knee-problems with either meniscus or the cruciate ligaments, hip-problems such as hip-dysplasis and legg Perthes, degenerative myelophatis, young dogs with growth problems, elder dogs suffering from decreased mobility, young sport-pigeons,...

### Experimental support (II)

In order to evaluate the positive effect of the nutritional supplement according to the present inventio on the recovery of bodily injuries or lesions with patients, a series of tests have been carried out.

Tests have been carried out on a population of seven dogs, all having osteoarthritis problems, and being subjected to an artroscopic operative surgery of the elbow or knee (TPLO - Tibial Plateau Levelling Osteotomy, or TTA - Tuberositas Tibiae Advancement). Some of these patients have been fed with tablets containing the nutritional supplement according to the invention during a period of four weeks, starting six weeks after the operation.

The effect of the inventive nutritional supplement on the recovery or healing process was evaluated in threeways:
1) A subjective assessment of the dog-owner by means of a LOAD (Liverpool Osteoarthritis in Dogs) evaluation tool;
2) a subjective assessment of the veterinary surgeon by means of a standardized COAST (Canine Osteoarthritis Staging Tool) evaluation tool;
3) an objective measurement by means of a pressure plate.

In order to enhance the reliability of the tests, a placebo group has been included as negative control. So the tests as performed represent a clinical, prospective and placebo-controlled pilot study.

The patients subject of this study are adult dogs, on which osteoarthritis has been diagnosed, and that have been treated in the course of 2019 by the University of Ghent, Faculty of veterinary sciences at Merelbeke, close to Ghent, Belgium. Patients that six weeks after the operative surgery still suffered from limping, were selected for the study.

Two subgroups were included in the study:
1) a first group of five dogs, to whom the inventive nutritional supplement was fed orally as a tablet;
2) a second group of two dogs, to whom placebo capsules were fed.

The dog-owners completed the LOAD-questionnaire at the occasion of a consult with the veterinary surgeon six weeks after the operative surgery, as well as twelve weeks after the operative surgery. At the first consult, they received the tablets, resp. the capsules from the surgeon to be fed during four weeks to their dogs by the owners. The owners are unaware of the fact whether their dogs will be fed with the inventive nutritional supplement or the placebo capsules. The placebo capsules contain lactose and a dyestuff but no active ingredient.

With respect to the feeding of the nutritional supplement during four weeks:
- during the first week, the dogs are fed with a double dose of the supplement;
- during the following three weeks, they are fed with a normal - maintenance - dose.

The dose takes into account the bodily weight of the dogs. The higher the weight, the higher (proportional) the amount of the nutritional supplement to be fed. Hereinafter is a schematic view of the procedure as followed in the tests:

| **Action:** | | **Evaluation:** |
|---|---|---|
| operative surgery | | pressure plate |
| | *six weeks after operative surgery:* | |
| first post-surgery control | | pressure plate + LOAD + COAST |
| start intake of inventive nutritional tablet or placebo capsules for 4 weeks | | |
| | *six weeks after first post-surgery control* | |
| second post-surgery control | | pressure plate + LOAD + COAST |

The inventive nutritional supplement was fed to the dogs during four weeks, once a day, in the form of a chewable tablet, preferably in the morning.

### Evaluation of the LOAD scores:

For the two dogs of the placebo control group, no improvement of het LOAD score was noted.

For the five dogs of the test group, an improved LOAD score was noted for two of the five patients.

### Evaluation of the COAST scores:

Contrary to the LOAD forms that were completed by the dog-owners, the COAST forms were completed by the veterinary surgeons.

For none of the two patients in the control group, an improved COAST stage was noted.

For two out of the five patients that were fed with the inventive nutritional supplement, the data for the COACH stage were incomplete; so these were not taken into account.

For the three remaining patients in the test group, an improved COAST stage was noted for one out of the three patients.

### Pressure Plate

With respect to the Mean Vertical Force, the Maximum Vertical Force, the Vertical Impact, and the Symmetry Index for the two patients of the control group, the results before and after the therapy were the same, or quite similar.

With respect to the above parameters for the five patients of the test group, an improvement of one or more pressure plate parameters were noted for all of the patients. For two patients an enhancement of the vertical ground reaction forces was noted and for three patients a decrease of the symmetry index could be observed.

Pressure plate measurements are, contrary to the above mentioned LOAD and COAST evaluations, objective data and as such are regarded as 'golden standards'. The pressure plate parameters that were evaluated in the study by the inventors are the Mean Vertical Force, the Maximum Vertical Force, the Vertical Impact and the Symmetry Index. These are all reliable parameters for the assessment of limping, according to Krotscheck et al., 2014, "Precision and accuracy of ground reaction force normalization in a heterogeneous population of dogs", Veterinary Surgery 43, 437-445.

### General Conclusion of the experimental data:

On the basis of the above described pilot study, a mild positive effect could be observed for the nutritional supplement according to the invention on the mobility of dogs suffering from osteoarthritis during the revalidation periode after an elbow or knee operative surgery.

## Claims

1. A nutritional supplement comprising the following components:
- Glutamine peptide;
- Lactoferrin;
- Milk salts;
- Tricalcium phosphate;
- Sodium acid pyrophosphate and
- Magnesium citrate.

2. The nutritional supplement according to claim 1, comprising the above components in the following amounts, expressed as weight parts:
- Glutamine peptide neutral from 500 to 600 parts of weight;
- Lactoferrin from 10 to 20 parts of weight;
- Milk salts from 450 to 550 parts of weight;
- Tricalcium phosphate from 50 to 150 parts of weight and
- Sodium acid pyrophosphate from 10 to 30 parts of weight.

3. The nutritional supplement according to any one of the aforementioned claims, further comprising one or more of the following components:
sodium bicarbonate, ferric gluconate, isomaltulose, dextrose, vanilla, wheat protein, vitamin C, collagen peptide, MSM, glucosamine and/or orange aroma.

4. The nutritional supplement according to any one of the aforementioned claims, further comprising one of more of following components:
chondroitin, hyaluronic acid, caffeine and/or taurine.

5. The nutritional supplement according to any one of the aforementioned claims, the milk salts consisting mainly of calcium and phosphor mineral compounds.

6. The nutritional supplement according to claim 5 whereby the calcium content is at least 25% and the phosphor content at least 10% of the total mineral content.

7. Nutritional supplement according to anyone of the claims 1-6 for use in the enhancement of the healing of injuries.

## Patentansprüche

1. Ein Nahrungsergänzungsmittel, das die folgenden Komponenten enthält:
- Glutaminpeptid;
- Laktoferrin;
- Milchsalze;
- Trikalziumphosphat;
- Natriumsaures Pyrophosphat und
- Magnesiumcitrat.

2. Das Nahrungsergänzungsmittel nach Anspruch 1, das die oben genannten Komponenten in den folgenden Mengen, ausgedrückt als Gewichtsteile, enthält:
- Glutaminpeptid neutral von 500 bis 600 Gewichtsteilen;
- Laktoferrin von 10 bis 20 Gewichtsteilen;
- Milchsalze von 450 bis 550 Gewichtsteilen;
- Trikalziumphosphat von 50 bis 150 Gewichtsteilen und
- Natriumsaures Pyrophosphat von 10 bis 30 Gewichtsteilen.

3. Das Nahrungsergänzungsmittel nach einem der vorgenannten Ansprüche, das außerdem eine oder mehrere der folgenden Komponenten enthält: Natriumbicarbonat, Eisengluconat, Isomaltulose, Dextrose, Vanille, Weizenprotein, Vitamin C, Kollagenpeptid, MSM, Glucosamin und/oder Orangenaroma.

4. Das Nahrungsergänzungsmittel nach einem der vorgenannten Ansprüche, das außerdem eine oder mehrere der folgenden Komponenten enthält: Chondroitin, Hyaluronsäure, Koffein und/oder Taurin.

5. Das Nahrungsergänzungsmittel nach einem der vorgenannten Ansprüche, wobei die Milchsalze hauptsächlich aus Calcium- und Phosphor-Mineralverbindungen bestehen.

6. Das Nahrungsergänzungsmittel nach Anspruch 5, wobei der Calciumgehalt mindestens 25 % und der Phosphorgehalt mindestens 10 % des gesamten Mineralstoffgehalts beträgt.

7. Das Nahrungsergänzungsmittel nach einem der Ansprüche 1 bis 6 zur Verwendung bei der Förderung der Heilung von Verletzungen.

## Revendications

1. Un supplément nutritionnel, comprenant les composants suivants:
- le peptide de glutamine
- le lactoferrine;
- les sels de lait;
- le phosphate de tricalcium;
- le pyrophosphate d'acide de sodium et
- le citrate de magnésium.

2. Le supplément nutritionnel selon la revendication 1, comprenant les composants précités dans les quantités suivantes, exprimé en part de poids:
- le peptide de glutamine neutre de 500 à 600 parts de poids,
- le lactoferrine de 10 à 20 parts de poids,
- les sels de lait de 450 à 550 parts de poids,
- le phosphate de tricalcium de 50 à 150 parts de poids et
- le pyrophosphate d'acide de sodium de 10 à 30 parts de poids.

3. Le supplément nutritionnel selon l'une quelconque des revendications précédentes, comprenant en plus un ou plusieurs des composants suivants:
le bicarbonate de sodium, le gluconate de fer, l'isomaltulose, le dextrose, la vanille, les protéines de blé, la vitamine C, le peptide de collagène, le MSM, le glucosamine et/ou l'arôme d'orange.

4. Le supplément nutritionnel selon l'une quelconque des revendications précédentes, comprenant en plus un ou plusieurs des composants suivants:
le chondroitine, l'acide d'hyalurone, la caféine et/ou la taurine.

5. Le supplément nutritionnel selon l'une quelconque des revendications précédentes, dans lequel les sels de lait consistent en grande partie de composés minéraux de calcium et de phosphore.

6. Le supplément nutritionnel selon la revendication 5, dans lequel le montant de calcium est au moins de 25 % et le contenu en phosphore est au moins de 10 % du contenu total en minéraux.

7. Le supplément nutritionnel selon l'une quelconque des revendications 1-6 pour usage dans l'amélioration de la guérison de blessures.
